# EUROPEAN PATENT APPLICATION

(11) **EP 3 926 039 A1**
(43) Date of publication of application: **22.12.2021**
(21) Application number: 20180558.7
(22) Date of filing: 17.06.2020
(51) Int. Cl.: C12N 1/08, C12N 9/22

(54) **USE OF A NUCLEASE FOR REDUCING THE VISCOSITY AND/OR PREVENTING AN INCREASE IN VISCOSITY OF A FERMENTATION BROTH**

(71) Applicant: AB Enzymes GmbH, 64293 Darmstadt (DE)
(72) Inventor: KAYA, Yasar, 64293 Darmstadt (DE); NEUBER, Stefania, 64293 Darmstadt (DE); SEEFRIED, Michael, 64293 Darmstadt (DE); HAARMANN, Thomas, 64293 Darmstadt (DE); LORENZ, Patrick, 64293 Darmstadt (DE); SCHWERDTFEGER, Ruth, 64293 Darmstadt (DE); WALLIS, Evelyn, 64293 Darmstadt (DE)
(74) Representative: Hiebl, Inge Elisabeth

(57) **Abstract**

The invention relates to the use of a protein having nuclease activity for reducing the viscosity and/or preventing an increase in viscosity of a fermentation broth, which comprises intact microorganisms after termination of the fermentation process. The invention moreover relates to a method for reducing the viscosity and/or preventing an increase in viscosity of a fermentation broth comprising intact microorganisms after fermentation and comprising a step of introducing a protein having nuclease activity into the fermentation broth during the fermentation until the start of recovering the end product. The invention further relates to fermentation processes comprising said use of a protein having nuclease activity to allow more flexibility to the downstream processing of the fermentation broth.

## Description

The present invention generally relates to the use of a protein having nuclease activity for reducing the viscosity and/or preventing an increase in viscosity of a fermentation broth. Further provided is a method for reducing the viscosity and/or for preventing an increase in viscosity of a fermentation broth after fermentation by introducing a protein having nuclease activity into the fermentation broth. Further provided are fermentation processes including a step comprising the introduction of a nuclease into a fermentation broth.

### BACKGROUND OF THE INVENTION

Microbial fermentations are used for the manufacture of a large number of pharmaceutical and industrial products including antibiotics, organic acids, amino acids, proteins, vitamins, polymers, enzymes, starter cultures etc. For such fermentation processes a number of different microorganisms may be used. Due to their high production capacity, secretion efficiency and metabolic versatility, filamentous fungi and bacteria are widely used as efficient cell factories in the production of various substances. One problem with cultivation broth containing microorganisms is the problem of highly viscous fermentations. The problem is particularly aggravated since fermentation broths from filamentous fungi and a number of bacteria tend to show an increased viscosity after the end of the fermentation, i.e. the fermentation broth either has to be processed immediately or efforts have to be taken to keep the viscosity of the fermentation broth low, for example by stirring the solution. This, however, may present a problem for large scale industrial production of substances since stirring a solution or suspension is time and energy consuming and may negatively influence the fermentation products due to an increased input of oxygen. Before the harvest of the product in a down-stream process via filtration or centrifugal separation, a flocculation step may be performed. In this step, cells and insoluble particles are agglomerated, whereas the liquid contains the protein of interest. During this step, viscosity may increase and hinder proper flocculation. Moreover, filtration steps are more difficult due to a high viscosity and the yield in the target product after purification will be lower due to an increased thickness of the fermentation broth. This is due to the blocking of filter sheets and therefore material loss as soluble proteins in the liquid, which cannot be filtrated properly. Problems with viscosity may arise also for downstream methods other than filtration. In centrifugal separation for instance, the protein fraction may not be separated properly, which may lead to yield loss and impurities in the product.

In the prior art, DNA is considered to be associated with a high viscosity of fermentation broths and it is proposed to treat said solution with DNases and RNases to improve purity and to reduce the high viscosity of said solutions (see, for example, Pharm. Bioprocess. (2016) 4(5), 095-099). US 9,796,994 proposes to remove contaminating nucleic acids or to digest these to such an extent that no further restriction of the following process steps of a fermentation occurs. It is proposed to break down the nucleic acids to such small fragments using nucleases that the viscosity of the samples is reduced and the resulting decomposition products can be separated using simple methods such as ultrafiltration.

WO 2015/166037 discloses a method for removing DNA from a fermentation broth comprising a protein of interest and a microorganism producing the protein of interest, whereby said method comprises heating the fermentation broth to a temperature of at least 70°C.

US 2018/0030087 discloses a method for removing residual DNA from a fermentation process comprising a protein of interest and a microorganism producing the protein of interest, whereby said method comprises adding a poly aluminium chloride to the fermentation broth and separating the flocculated microorganism as well as exogenous DNA from the fermentation broth.

WO 2012/145598 discloses strains of filamentous fungi having genetic alterations that give rise to an altered viscosity phenotype. In a particular embodiment, said variant strains do not produce the Crz1protein. The gene *crz1* encodes a calcineurin-regulated transcription factor which is dephosphorylated when the phosphatase calcineurin is activated by Ca²⁺/calmodulin. It then enters the nucleus and induces expression of a number of genes, many of which encode proteins with cell wall-related functions (Yoshimoto *et al.* 2002; Lagorce *et al.* 2003; Garcia *et al.* 2004; Karababa *et al.* 2006; Pardini *et al.* 2006). Deletion of *crz1* or a homolog can result in alterations in hyphal morphology (Kothe, G. and Free, S. 1998, Prokisch, H. *et al*.1997). Crz1 is associated with altered morphology and it is believed that the alteration of *crz1* expression and/or activity in filamentous fungi can alter the cell wall, thereby producing a more compact cellular morphology characterized by shorter hyphae and a more yeast-like appearance. Other genes associated with altered morphology and mentioned in the patent are *sfb3*, *seb1, mpg1, gas1,* and *tps2.*

In summary, this means that according to the prior art, an increase in the viscosity of a fermentation broth is at least partly ascribed to larger amounts of nucleic acids and various proposals have been made to arrive at fermentation broths having a lower content of DNA or RNA. All approaches of the prior art, however, are based on the assumption that DNA/RNA is the main factor for increasing the viscosity of a fermentation broth and that DNA/RNA is mainly introduced into the fermentation broth by lysis of the cells. The prior art, however, does not address the problem of an increase in viscosity of a fermentation broth after the fermentation has been terminated, *i.e.* when the fermentation batch is left standing. Moreover, the approaches of the prior art only address viscosity problems of fermentation broths which are due to an increased amount of DNA and/or RNA, but do not consider the viscosity of a fermentation broth independent of significant extracellular DNA/RNA content increase. A significant extracellular DNA/RNA content increase is usually due to the lysis of the cells of the fermented microorganism at the end of the fermentation.

Hence, it is an object of the present invention to provide a method for reducing the viscosity and/or preventing an increase in viscosity of a fermentation broth after the fermentation process has been terminated. The method of the invention is to reduce the thixotropy of a fermentation broth and is, moreover, to provide more flexibility to the downstream processing of the fermentation product or the fermentation broth by avoiding an increase in the viscosity during intermediate steps between end of fermentation and cell separation. The object of the invention is in particular to reduce the viscosity and/or prevent an increase in viscosity of a fermentation broth obtained from a fermentation process which does not necessitate a lysis of the cells, *p. ex.* after which either the cells as such or the fermentation broth is further processed.

### SUMMARY OF THE INVENTION

The inventors have discovered that the addition of a protein having nuclease activity to a fermentation broth, comprising intact microorganisms avoids an increase of the viscosity of said broth and/or prevents an increase in the viscosity of said fermentation broth after termination of the fermentation process. It has been found that said effect is independent of significant DNA increase in the fermentation broth, *i.e.* said effect is also evident when the cells are not lysed so that only minor and technically unavoidable amounts of DNA or RNA have been released into the fermentation medium. Said viscosity increase could be only observed in the presence of intact cells. It has moreover been found that slime which has already been formed in a fermentation broth can be dissolved by the addition of a nuclease. This means that the above use of a protein having nuclease activity is particularly suitable for fermentation processes at the end of which the cells of the microorganism are not lysed but remain intact. The above use avoids an increase in viscosity or reduces the viscosity after termination of the fermentation process.

Hence, the present invention relates to the use of a protein having nuclease activity for reducing the viscosity and/or preventing an increase in viscosity of a fermentation broth which comprises intact microorganisms after termination of the fermentation process. The invention further relates to a method for reducing the viscosity and/or preventing an increase in viscosity of a fermentation broth comprising intact microorganisms after termination of the fermentation process, comprising the step of introducing a protein having nuclease activity into the fermentation broth before recovering the end product. The invention, moreover, relates to fermentation processes introducing a protein having nuclease activity before recovering the end product.

The process of producing a target product usually comprises three phases, i.e. a first phase is the actual fermentation process, the second phase is the time between the completion or termination of the fermentation process and the further processing of the fermentation broth which time can last for a longer period and usually can last until two weeks and the final phase is the phase of the further processing of the fermentation broth, *i.e.* the recovery of the final product. This can be, for example, the filtration of liquid and solid particles (supernatant and cells) or the further pumping of the whole broth. The protein having nuclease activity has to be added while the fermentation broth is still liquid. Hence, the protein having nuclease activity is added at latest before recovering the end product. This means that the protein having nuclease activity can be added during any time of the fermentation process until the start of recovering the end product. Preferably, the protein having nuclease activity is added after termination of the fermentation process until the start of recovering the end product. More preferably, the protein having nuclease activity is added after the termination of the fermentation process and at least ten minutes before the start of recovering the end product. More preferably, the protein having nuclease activity is added one hour before the start of recovering the end product and at least ten minutes before the start of recovering the end product.

The present invention may be useful for any production processes in industrial scale which need a cell separation step. That includes fermentation processes based on minimal media or complex media comprising complex nitrogen and carbon sources. The fermentation could be set up as a batch, a repeated batch, a fed-batch, a repeated fed-batch or a continuous fermentation process, whereas a fed-batch process is preferred. The fermentation processes for which the use of the invention may favourably be applied are basically any fermentation processes in the art which use microorganisms which by nature or by recombinant modification produce an organic molecule such as enzymes, vitamins, hormones, drugs etc. At the end of a fermentation process the fermentation broth usually has to be downstream processed for isolating the microorganism and / or a target product produced by the microorganism.

### DETAILED DESCRIPTION OF THE INVENTION

The present invention relates to a method for reducing the viscosity and/or preventing an increase in viscosity of a fermentation broth comprising intact microorganisms after termination of fermentation comprising the step of introducing a protein having nuclease activity into said fermentation broth before the start of recovering the end product. The invention, moreover, also relates to the use of a protein having nuclease activity for reducing the viscosity and/or preventing an increase in viscosity of a fermentation broth comprising intact microorganisms after termination of the fermentation process.

As outlined above the microorganisms remain intact after the end of the fermentation process.

As used herein, the term "intact microorganism" means that the microorganism is not subject to intended lysis or disruption by *e.g.* detergents, mechanical or enzymatical disruption leading to release of intracellular ribonucleic acids (RNA) and deoxyribonucleic acids (DNA).

The inventors have discovered, that by adding a protein having nuclease activity to a fermentation broth containing intact microorganisms leads to a surprising reduction of the viscosity and/or prevents an increase in viscosity of that microorganism containing fermentation broth. Without the addition of a protein, having nuclease activity such a fermentation broth which may be in the form of a suspension tends to increase in viscosity when the fermentation broth is left standing for several hours or days, so that the down-stream processing becomes difficult or even impossible.

The protein having nuclease activity may be added during any stage of the fermentation process until the process of recovering the end product (separation of the cells) or before any downstream process has started, e.g. at the beginning, during, after, at the end of the fermentation process, or before the start of recovering the end product. The protein having nuclease activity may also continuously be added by production through a microorganism in the fermentation broth. Preferably the protein having nuclease activity is added at the end of the fermentation process or after the termination of the fermentation process until the start of recovering the end product.

That use of a nuclease is basically applicable to a variety of fermentation processes typically used for the industrial production of organic substances such as enzymes, vitamins, hormones, drugs, polymer precursors or other technically useful products. The invention is of course particularly suitable for fermentation processes at the end of which the fermentation broth as such is further processed or at the end of which intact microorganisms are to be separated for example by centrifugation techniques such as decanter centrifugation, continuous flow centrifugation or disc stack separation or filtration techniques such as microfiltration, ultrafiltration etc.. Particularly, preferred fermentation processes for which the invention is applicable are batch, repeated batch, fed-batch, repeated fed-batch, or continuous fermentation processes.

As used herein, the term "viscosity" has its general meaning in the art. It refers to the measurement of the resistance of fluid which is being deformed by either shear stress or tensor stress. Viscosity is usually expressed in Pa·s. Typically, the viscosity of a fermentation broth is measured by a flow measurement assay as described in the examples below. Other techniques for determination of viscosity next to applying rotational viscosimeters are systems based on capillary viscosimeters, falling sphere viscosimeters and Doppler velocimeters. Viscosity can also be measured online in stirred tank bioreactors by applying the above mentioned methods in a fermenter bypass as well as by measuring power input (torque sensor) or heat transfer capacities from the bulk medium to the jacket of the fermenter. (Schelden *et. al.* 2014).

Nucleases and genes encoding nucleases which are suitable for use in the method of the present invention can be obtained from a number of sources and may be *p. ex.* bacterial nucleases or fungal nucleases. They may be exonucleases, endonucleases, deoxyribonucleases or ribonucleases. Nucleases are expressed by a broad range of (micro)organisms and many of the corresponding genes have been cloned and characterized. Sources of nuclease genes include *Serratia marcescens* (GenBank Acc. No. M19495), *Shewanella oneidensis* MR-1 (GenBank Acc. No. AAN54138), *Anabaena* PCC7120 (GenBank Acc. No. X64706), *Bacillus subtilis* (GenBank Acc. No. U66480), *Staphylococcus hyicus* (GenBank Acc. No. L23973), *Staphylococcus intermedius* (GenBank Acc. No. X67678), *Escherichia coli* (GenBank Acc. No. X55818), *Shigella flexneri* (GenBank Acc. No. U30471), *Methanobacterium thermoautotrophicum* (GenBank Acc. No. AE000833), and *Methanococcus jannaschii* (GenBank Acc. No. U67584). Some of these nucleases are also available as commercial products, e.g., DNase I from bovine/human origin, nuclease from *Staphylococcus aureus,* and nuclease from *Serratia marcescens* (Benzonase). Preferably, the nuclease cleaves both ribonucleic acid (RNA) and deoxyribonucleic acid (DNA). The nuclease preferably is active over a wide temperature and pH range. Moreover, the nuclease is also preferably tolerant to the presence of processing additives such as salts, surfactants and stabilizers. According to the present invention, the following nucleases may advantageously be used: NucB from *Bacillus amyloliquefaciens,* as produced in *Bacillus pumilus.* NucB- is highly conserved in the genus *Bacillus* and orthologous proteins can be found in many other related organisms. This includes all proteins that preferably belong to the Pfam family PF14040 (Deoxyribonuclease NucA/NucB).

A protein having nuclease activity is preferably selected from bacterial nucleases, preferably bacterial nucleases belonging to the Pfam family PF14040, more preferably NucB or NucA from the group comprising Gram-positive bacteria such as a *Bacillus, Brevibacillus, Clostridium, Enterococcus, Geobacillus, Lactobacillus, Lactococcus, Lysinibacillus, Oceanobacillus, Paenibacillus, Staphylococcus, Streptococcus, Streptomyces,* or *Thermoactinomyces* or a Gram-negative bacteria such as a *Acinetobacter, Agrobacterium, Burkholderia, Enterobacter, Erwinia, Escherichia, Lysobacter, Methylomonas, Mesorhizobium, Photobacterium, Pseudomonas, Rhizobium, Serratia,* or *Xenorhabdus,* such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis.*

Another preferred embodiment is a nuclease of a filamentous fungus, preferably from *Aspergillus* or *Trichoderma,* more preferably from *Aspergillus oryzae* or *Trichoderma reesei.* A further preferred embodiment is a nuclease from *Bacillus* preferably from *Bacillus licheniformis, Bacillus amyloliquefaciens* or *Bacillus pumilus.*

According to the present invention, the nuclease may be added in a separate dosing during or after the end of the fermentation process. Separate dosing could mean that the nuclease is produced in a separate fermentation process by a microorganism or isolated from samples that naturally contain nucleases and supplemented to the fermentation process. This can be done at all stages of the fermentation process and afterwards.

This means that after termination of the fermentation process the addition of a nuclease has the above effect.

As a second option nucleases can be (co-)expressed by the organism of interest or by co-cultivation with an additional nuclease producing strain. The nuclease encoding gene can be introduced into the production strain's chromosome or on a self-replicating element (plasmid). The nuclease is preferably added to the fermentation process at a final concentration of at least 10 mg, at least 1 mg, at least 0.1 mg, at least 0.01 mg, at least 0.001 mg, at least 0.0001 mg nuclease per 100 ml fermentation broth.

The term "co-expressed" or "co-expression" of the protein having nuclease activity means introduction of a gene for expression of said protein into the microorganism capable of producing the target protein or the microorganism of interest.

Preferred concentration ranges are 0.0001 mg to 10 mg per 100 ml fermentation broth, preferably 0.001 to 10 mg per 100 ml fermentation broth, more preferably 0.01 to 10 mg per 100 ml fermentation broth, still more preferably 0.1 mg to 10 mg per 100 ml fermentation broth, still more preferably 1 mg to 10 mg per 100 ml fermentation broth. Further preferred ranges are 0.0001 mg to 1 mg per 100 ml fermentation broth, 0.001 to 1 mg per 100 ml fermentation broth, 0.01 to 1 mg per 100 ml fermentation broth, still more preferably 0.1 to 1 mg per 100 ml fermentation broth. A particularly preferred range is a concentration of 0.35 to 3.5 mg nuclease per 100 ml fermentation broth. In case the nuclease is produced directly within the fermentation broth, a person skilled in the art can easily check the appropriate concentration and can adapt the in situ produced nuclease concentration appropriately, for example by dilution or concentration of the fermentation broth.

Appropriate nucleases and respective nuclease concentrations which are suitable for use in the method of the present invention can be selected by those skilled in the art with minimal experimental efforts.

The use or the method of the present invention is suitable for any fermentation in industrial scale. The microorganism may be fermented by any method known in the art. In an industrial fermentation a protein of interest is produced by a classical or genetically modified organism in a bioreactor. A fermentation also usually comprises one or more preculture steps for propagation of the strain with a distinct medium as well as incubation time and conditions adapted to the strain and protein of interest. The production bioreactor is inoculated with a certain amount of the last preculture step. With optimization of the fermentation medium and the preculture conditions, fermentation yield can be significantly increased. An example for fermentation and fermentation media are given in EP 2145006B1.

The fermentation is generally performed as a batch or continuous fermentation process. Preferably the fermentation is a batch, repeated batch, a fed-batch, a repeated fed-batch, or a continuous fermentation process.

The microorganism contained in the fermentation broth may be any microorganism that is to be further used as such or that is useful for producing an industrially relevant product as outlined above. The microorganism is preferably selected from filamentous fungi, yeast or bacteria comprising but not limited to the group of *Actinoplanes, Agrobacterium, Bacillus, Brevibacillus, Clostridium, Enterococcus, Escherichia, Erwinia, Geobacillus, Haemophilus, Lactobacillus, Lactococcus, Lysinibacillus, Oceanobacillus, Paenibacillus, Proteus, Pseudomonas, Rhizobium, Staphylococcus, Streptococcus, Streptomyces, Thermoactinomyces, Xanthomonas, Acremonium, Aspergillus, Aureobasidium, Bjerkandera, Ceriporiopsis, Chrysosporium, Coprinus, Coriolus, Cryptococcus, Filibasidium, Fusarium, Humicola, Kluyveromyces, Magnaporthe, Mucor, Myceliophthora, Neocallimastix, Neurospora, Paecilomyces, Penicillium, Phanerochaete, Phlebia, Pichia, Piromyces, Pleurotus, Saccharomyces, Schizophyllum, Streptomyces, Talaromyces, Thermoascus, Thielavia, Tolypocladium, Trametes,* or *Trichoderma.*

The microorganism is preferably selected from *Hyprocrea jecorina, Trichoderma reesei, Trichoderma viride, Trichoderma harzianum, Trichoderma koningii, Trichoderma longibrachiatum, Aspergillus niger, Aspergillus oryzae, Aspergillus awamori, Aspergillus fumigatus, Aspergillus foetidus, Aspergillus japonicus, Aspergillus nidulans, Humicola insolens, Humicola grisea, Streptomyces sp., Streptomyces violaceoruber, Bacillus licheniformis, Bacillus amyloliquefaciens, Bacillus pumilus, Bacillus subtilis, Bacillus sp, Bacillus megaterium, Geobacillus stearothermophilus, or Thermotoga maritima.*

According to one embodiment of the invention at least the following steps are encompassed:
(i) cultivating a microorganism of interest or a microorganism capable of producing a target product,
(ii) adding a nuclease by direct addition or by co-expression of the nuclease by the microorganism of interest or the microorganism capable of producing a target product
(iii) obtaining a fermentation broth comprising a nuclease and a) the microorganism of interest or b) a microorganism capable of producing target product and the target product, and
(iv) recovering a) the microorganism of interest or b) the target product.

According to a further embodiment at least the following steps are encompassed:
(i) cultivating a microorganism of interest or a microorganism capable of producing a target product,
(ii) obtaining a fermentation broth comprising a microorganism of interest or the target product and the microorganism capable of producing a target product,
(iii) adding a nuclease to the fermentation broth, and
(iv) recovering the microorganism or the target product.

According to a further embodiment at least the following steps are encompassed:
(i) cultivating a microorganism of interest or a microorganism capable of producing a target product, and co-cultivating a microorganism capable of producing a nuclease,
(ii) obtaining a fermentation broth comprising a microorganism capable of producing a nuclease and a nuclease, and a) a microorganism of interest or b) a target product and the microorganism capable of producing a target product, and
(iii) recovering a) the microorganism of interest or b) the target product.

At the end of a fermentation process the fermentation broth usually has to be downstream processed for isolating the microorganism of interest and / or a target product produced by the microorganism. This can be done by flocculation followed by several filtration steps. One option would be starting with a pressurized plate frame filter or chamber filter press followed by subsequent depth filtration steps with different retention characteristics. Usually depth filtration is characterized by charge-related adsorption during filtration, and one may start with a filter medium which possesses a nominal retention range larger than 0.5 µm, and end with a filter medium with a nominal retention range of 0.2-0.4 µm (sterile filtration). Other options would be centrifugal separations via a decanter centrifuge (for larger particles) or a disc stack separator. Due to the centrifugal force, the solid particles separate out in the disc stack and slide on the underside of the discs into the solids chamber of the bowl. The disc stack is used to separate the product stream into many partial streams. This reduces the sedimentation path of the solid particles. Due to the high centrifugal force, even finer particles can settle out.

The term "start of recovering the microorganism or the target product" or "start of recovering the end product" means the separation of cells from the broth.

As outlined above, fermentation broths tend to increase in viscosity at the end of the fermentation upon standing even in the presence of intact microorganisms. An increased viscosity of the solution makes all further downstream processing steps more difficult and filtration steps in particular more difficult. Hence, the yield of the target product or the microorganism as such will be lower.

According to the invention, it has surprisingly been found that by directly adding a nuclease to the fermentation broth of a fermentation effected preferably by a filamentous fungus or a *Bacillus,* no further increase in viscosity is observed and already formed slime will be dissolved by addition of a nuclease and a homogenous suspension may be obtained again. It has particularly been found that the fermentation broth of *Bacillus* and *Aspergillus* fermentations starts to form a cell suspension with high viscosity within a few hours after termination of the fermentation and termination of the stirring, unless cells and supernatant are immediately separated. Once a high viscosity solution has been formed, a separation of cells and supernatant is no longer possible. The viscosity could only be reduced by restarting the stirring (thixotropic effect). Since, however, every production process necessarily involves a standing time of several hours, such an increase in viscosity is to be avoided. Avoiding an increase in viscosity after termination of the fermentation process will therefore provide more flexibility to the production process, i.e., the fermentation broth may be left standing for several hours or days/weeks before processing.

Without wishing to be bound, it is assumed that the increase in viscosity is due to an interaction of DNA and cells and not only due to the presence of DNA, such as, for example, has been described for *E. coli* cell lysates (EP 1068294). The reason therefor is that in supernatants of the fermentations no significantly increased DNA concentrations (comparable to cell lysates of *E. coli*) could be observed and an increased viscosity was only observable in the presence of cells. Since in the above mentioned cases the suspension is a thixotropic suspension, it can be concluded that the lysis of the cells after termination of the fermentation has either no or only a minimal effect on the development of a viscous suspension.

It has further been observed that for fermentations involving *Aspergillus* species, an increased viscosity of the fermentation broth already occurs during fermentation. To check whether nucleases can improve the properties of the fermentation broth for this type of fermentation, experiments were carried out whereby the fermentation broth was left standing for several hours. It could be shown that the fermentation broth also shows a thixotropic character which can consistently be removed by addition of a nuclease, preferably a *Bacillus amyloliquefaciens* NucB nuclease, as produced in *B. pumilus.* Thixotropy is defined as a time-dependent shear thinning property, whereas viscosity is measured in Pascal seconds (Pa·s) and defines the resistance of fluids to gradual deformation by external physical stressors.

The target product may be a microorganism of interest or any protein, such as, for example, food enzymes, feed enzymes, technical enzymes, hormones, immunoglobulins, vaccines, antibacterial proteins or antiviral proteins, or any other compound, such as amino acids, sugars, vitamins, antibiotics, solvents or any other metabolite produced by the microorganism and whereas target product produced by the microorganism may be secreted or not secreted. A microorganism of interest may be any microorganism (wild-type or genetically modified) that is to be produced in larger quantities by a fermentation process.

### DESCRIPTION OF THE FIGURES

The following figures explain the subject-matter of the present invention.

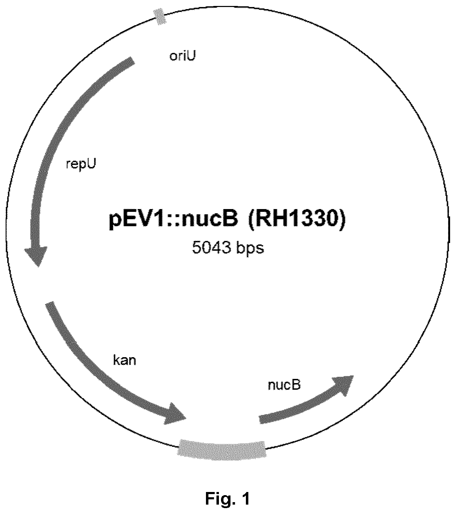

Figure 1 shows a plasmid for the expression of the NucB nuclease from *B. amyloliquefaciens.*

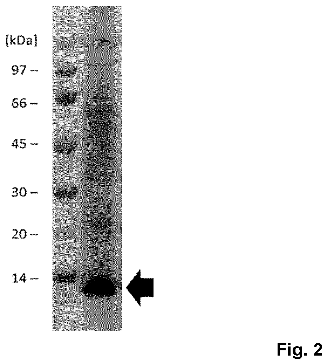

Figure 2 shows an SDS-PAGE analysis of the supernatant of the fermentation of strain *B. pumilus* RH11925. The protein band of nuclease NucB was detectable at 11 kDa and is marked by an arrow. Amersham Low Molecular Weight Calibration Kit (GE Healthcare) was used for molecular weight determination.

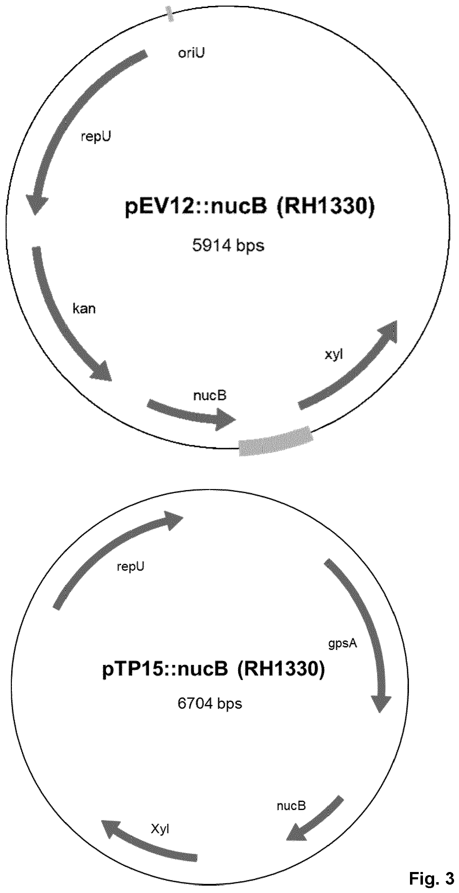

Figure 3 shows two plasm ids for co-expression of the nuclease NucB from *B.* am*yloliquefaciens* with xylanase as a model enzyme in *B. pumilus* (pEV12::nucB) and *B. amyloliquefaciens* (pTP15::nucB).

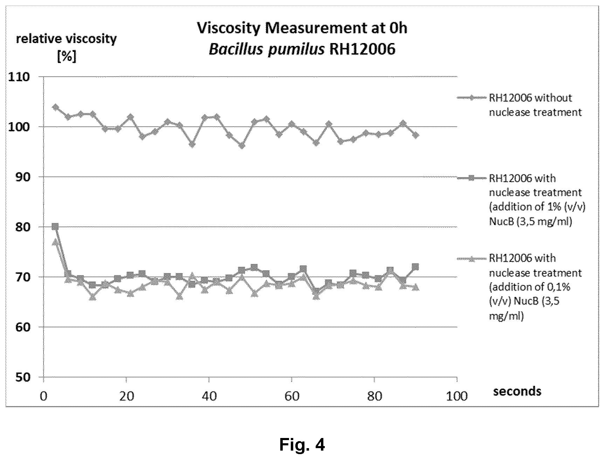

Figure 4 shows the measurement of the viscosity of a fermentation broth of *Bacillus pumilus* strain RH12006 after a standing time of 0 hours. The fermentation broth was treated either with 0.1 or 1 % (v/v) NucB (3.5 mg/ml) and compared to the untreated reference. Viscosity was measured with a constant stirring rate of 100 rounds per minute, 20 points of measurement per minute and a duration of measurement of 3 seconds. The untreated reference was set to 100 % and used to calculate the relative viscosity.

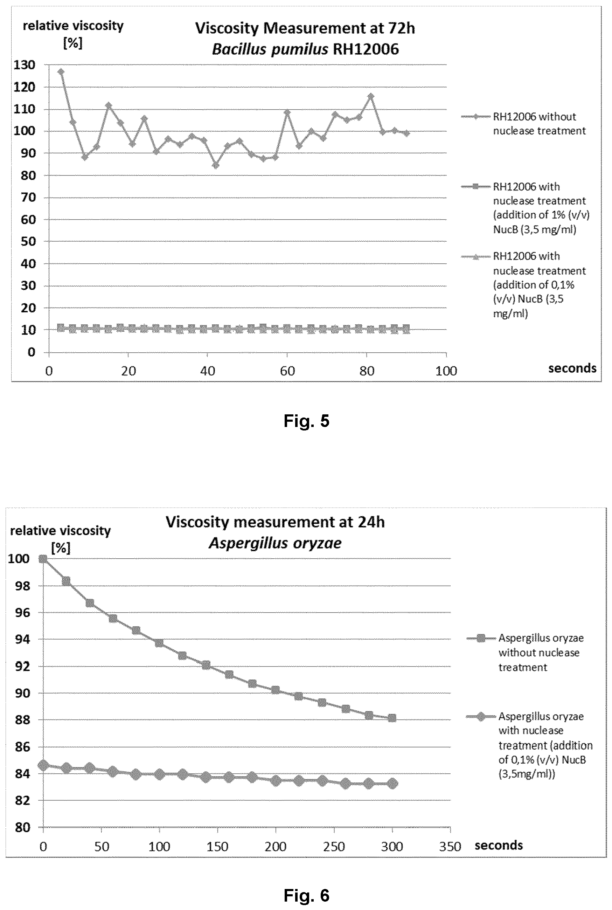

Figure 5 shows the measurement of the viscosity of the fermentation broth of *Bacillus pumilus* strain RH12006 after a standing time of 72 hours. The fermentation broth was treated either with 0.1 or 1 % (v/v) NucB (3.5 mg/ml) and compared to the untreated reference. Viscosity was measured with a constant stirring rate of 100 rounds per minute, 20 points of measurement per minute and a duration of measurement of 3 seconds. The untreated reference was set to 100 % and used to calculate the relative viscosity.
Figure 6 shows the measurement of the viscosity of samples from the fermentation broth of an *Aspergillus oryzae* fermentation producing alpha amylase after a standing time of 24 hours. The fermentation broth was treated with 0.1 % (v/v) NucB (3.5 mg/ml) and compared to the untreated reference. Viscosity was measured with a constant stirring rate of 100 rounds per minute, 3 points of measurement per minute and a duration of measurement of 5 seconds. The starting point of the untreated reference was set to 100 % and used to calculate the relative viscosity.

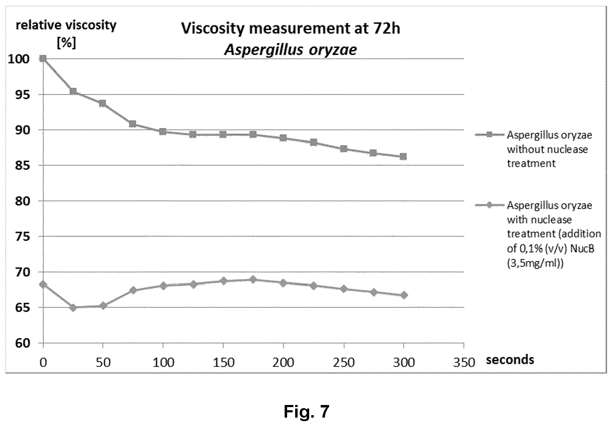

Figure 7 shows the measurement of the viscosity of samples from the fermentation broth of an *Aspergillus oryzae* fermentation producing alpha amylase after a standing time of 72 hours. The fermentation broth was treated with 0.1 % (v/v) NucB (3.5 mg/ml) and compared to the untreated reference. Viscosity was measured with a constant stirring rate of 100 rounds per minute, 3 points of measurement per minute and a duration of measurement of 5 seconds. The starting point of the untreated reference was set to 100 % and used to calculate the relative viscosity.

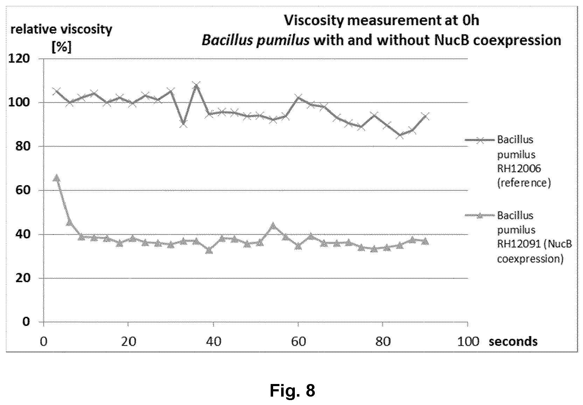

Figure 8 shows the measurement of the viscosity of the fermentation broth of *Bacillus pumilus* strain with (RH12091) and without nuclease NucB coexpression (RH12006) after a standing time of 0 hours. Viscosity was measured with a constant stirring rate of 100 rounds per minute, 20 points of measurement per minute and a duration of measurement of 3 seconds. The starting point of the untreated reference was set to 100 % and used to calculate the relative viscosity.

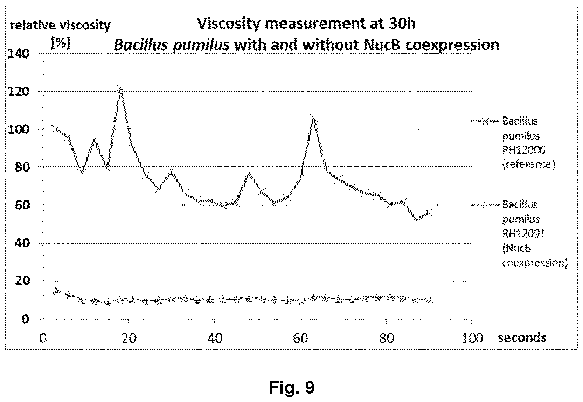

Figure 9 shows the measurement of the viscosity of the fermentation broth of *Bacillus pumilus* strain with (RH12091) and without nuclease NucB coexpression (RH12006) after a standing time of 30 hours. Viscosity was measured with a constant stirring rate of 100 rounds per minute, 20 points of measurement per minute and a duration of measurement of 3 seconds. The starting point of the untreated reference was set to 100 % and used to calculate the relative viscosity.

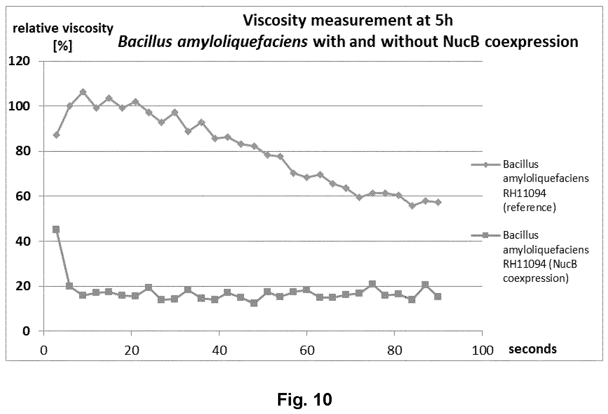

Figure 10 shows the measurement of the viscosity of the fermentation broth of *Bacillus amyloliquefaciens* with (RH12111) and without nuclease NucB coexpression (RH11094) after a standing time of 5 hours. Viscosity was measured with a constant stirring rate of 100 rounds per minute, 20 points of measurement per minute and a duration of measurement of 3 seconds. The starting point of the untreated reference was set to 100 % and used to calculate the relative viscosity.

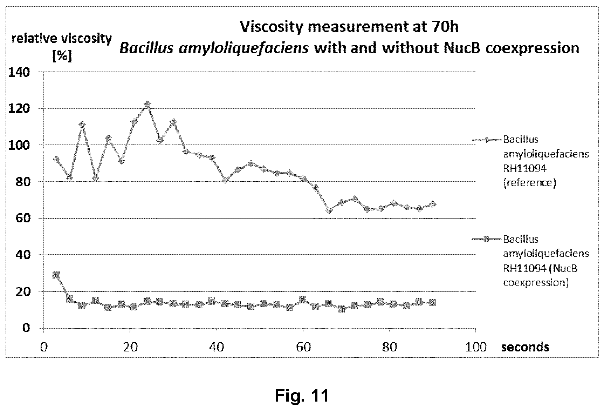

Figure 11 shows the measurement of the viscosity of the fermentation broth of *Bacillus amyloliquefaciens* with (RH12111) and without nuclease NucB coexpression (RH11094) after a standing time of 70 hours. Viscosity was measured with a constant stirring rate of 100 rounds per minute, 20 points of measurement per minute and a duration of measurement of 3 seconds. The starting point of the untreated reference was set to 100 % and used to calculate the relative viscosity.

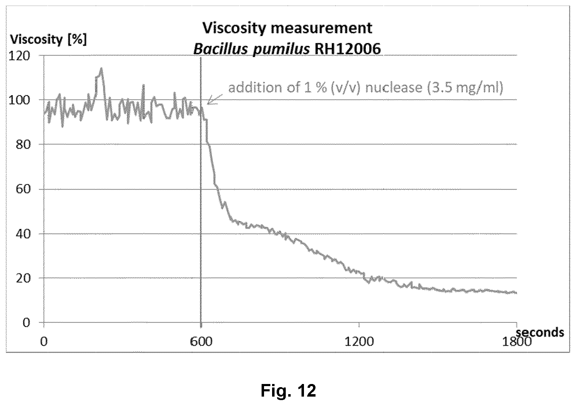

Figure 12 shows the change in viscosity of slime formed after the fermentation of *Bacillus pumilus* strain RH12006 before and after treatment with nuclease. 1% (v/v) of Nuclease NucB (3.5 mg/ml) was added to the fermentation broth after 10 minutes. Viscosity was measured with a constant stirring rate of 100 rounds per minute, 8 points of measurement per minute and a duration of measurement of 7.5 seconds. The baseline before addition of the nuclease NucB (timeframe 0 - 600 sec) was set to 100 % and used to calculate the relative viscosity.

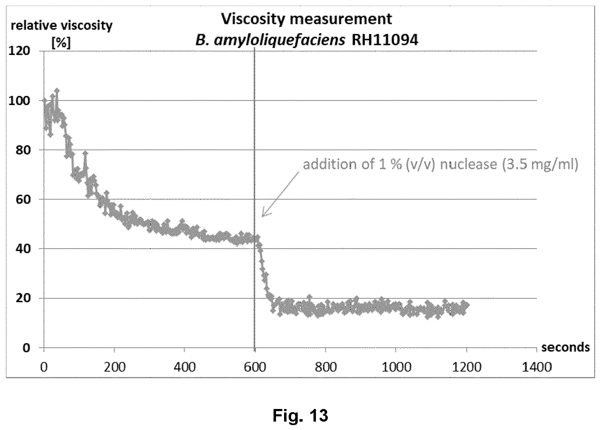

Figure 13 shows the change in viscosity of the fermentation broth of *Bacillus amyloliquefaciens* strain RH11094 before and after treatment with nuclease NucB. 1% (v/v) of Nuclease NucB (3.5 mg/ml) was added to the fermentation broth after 10 minutes. Viscosity was measured with a constant stirring rate of 100 rounds per minute, 8 points of measurement per minute and a duration of measurement of 7.5 seconds. The baseline before addition of the nuclease NucB (timepoints 0 -600 sec) was set to 100 % and used to calculate the relative viscosity.

The following examples explain the invention further.

### EXAMPLES

### Example 1: Construction of a strain for expression of a nuclease

Two types of plasmids have been prepared. The first plasmid type is a plasmid in which a NucB variant (from *B. amyloliquefaciens*) is overexpressed in a separate fermentation process. The second plasmid type is a plasmid for the co-expression of a nuclease activity together with a target product (xylanase).

### 1.1 Cloning of a plasmid for the expression of a nuclease

The *nucB* gene from *B*. *amyloliquefaciens* was placed under the control of the PaprE promotor from *B. licheniformis* DSM13. As a signal peptide the naturally occurring signal peptide was used. The cloning was effected by means of a PCR amplification and a consequent Gibson assembly using the following primers. Backbone and insert were amplified with the listed primers using the Phusion^{®} High-Fidelity DNA Polymerase (New England Biolabs). Purification of the resulting PCR products was performed using the Wizard^{®} SV Gel & PCR Clean-Up Kit (Promega). Assembly of the purified backbone and insert was subsequently achieved with the NEBuilder^{®} HiFi DNA Assembly Cloning Kit (New England Biolabs).

**Table 1.**

| **name of the primer** | **sequence 5'→ 3'** | **Tm** | **target fragment** |
|---|---|---|---|
| pEV1_1 | GACAGAGATATACCGACAGTG | 58 | pEV1 vector backbone |
| pEV1_2 | TACTCACTCTCCTCCTTTTTATTC | 58 | |
| nucB_fw1 | GGAGGAGAGTGAGTAATGAATGCGTTTATGAAATGGGCGGC | 73 | Insert _nucB |
| nucB_rv | CGGTATATCTCTGTCTTACTGAACGATAAATAATACTCTCGTGCCG | 72 | |

The cloning batch was subsequently transformed into a supercompetent *Bacillus subtilis* strain (SCK6; *Zhang* & *Zhang 2010*). The thus constructed expression plasmid was checked as to its integrity by means of a plasmid preparation, restriction enzyme digest and Sanger sequencing. Plasmid isolation was done from a fresh overnight culture in Luria Bertani medium with the aid of the QIAprep Spin Miniprep Kit (QIAGEN). Restriction enzyme digest was performed and evaluated via gel electrophoresis. Sanger sequencing was performed externally by SeqLab (Microsynth AG). The plasmid was then transformed into a *B. pumilus* strain resulting in the nuclease overexpressing strain RH11925. The thus obtained clones were verified via plasmid preparation, restriction enzyme digest and Sanger sequencing and three clones were further cultivated in shake flasks. The plasmid map of the created construct is shown in Figure 1.

### 0.5 L MBR (Mini BioReactor) fermentation

The strain RH11925 was then fermented in a scale of 0.5 L MBR in a standard fermentation process as described in EP2145006 B1.

After termination of the fermentation the culture broth was harvested and centrifuged. The supernatant was sterile filtered and stored at -20°C for further use. Additionally, the supernatant was analysed by means of a qualitative SDS-PAGE. Amersham Low Molecular Weight Calibration Kit (GE Healthcare) was used for molecular weight determination. The results are presented in Figure 2. The size of the nuclease NucB was determined as 11 kDa. To verify that samples contain active Nuclease NucB spiking experiments were carried out with purified plasmid DNA. 150 ng of plasmid DNA were spiked into 5 µl of supernatant and incubated for 30 min at room temperature. Finally, the samples were mixed with 10x loading buffer and analyzed by agarose gel electrophoresis. Plasmid DNA spiked into supernatant of strain *B. pumilus* RH11925 was fully digested after 30 min, whereas plasmid DNA in supernatant of the reference strain *B. pumilus* RH11689 still remained intact after 30 min. Total protein (Bio-Rad Protein Assay) was measured in the supernatant of the fermentation sample from *B. pumilus* RH11925. The concentration of the nuclease NucB was determined with 3.5 mg/ml.

### 1.2 Cloning of the plasmids for the co-expression of a nuclease

The nuclease NucB of *B. amyloliquefaciens* as used under item 1.1 was further cloned together into the xylanase expression plasmids pEV12 (EP 3385377 A1) and a derivative of pTP15 (EP2145006 B1). The corresponding plasmids are shown in Figure 3. The cloning was effected by means of PCR amplification and a consequent Gibson assembly as detailed above using the following primers:

**Table 2.**

| **Name of the primer** | **Sequence 5'→3'** | **Tm** | **target** |
|---|---|---|---|
| pEV_1 | GTTCAAAATGGTATGCGTTTTG | 49 | Backbone |
| pEV_2 | TCGGCAAAAAATGATCTC | | |
| 1330_nucB_fw | ACGCATACCATTTTGAACTTTATCTGCCGAAGA- CACTG | 51 | Nuclease with promotor |
| 1330_nuCB_rv | GAGATCATTTTTTGCCGACGAACCGGA- GAAGTTACTG | | |

The construction of the plasmids and of the expression strains was carried out as described in section 1.1 above. The following strains were constructed and used for fermentation and standing time experiments (Table 3).

**Table 3**

| Species | Strain | Plasmid |
|---|---|---|
| *Bacillus pumilus* | RH12006 | pEV12 |
| *Bacillus pumilus* | RH12091 | pEV12::nucB |
| *Bacillus amyloliquefaciens* | RH11094 | pTP15 |
| *Bacillus amyloliquefaciens* | RH12111 | pTP15:nucB |

### Example 2: Fermentation for producing enzyme containing fermentation broth

### 1. Fermentations for separate nuclease treatment

### a. Bacillus pumilus fermentation (Xylanase):

The xylanase producing strain *B. pumilus* RH12006 was cultivated in a 30 L fermenter in a standard fermentation process as described in EP2145006 B1.

The fermentation broth obtained at the end of the fermentation was used for the determination of the viscosity with and without addition of nuclease NucB.

### b. Aspergillus oryzae fermentation (Amylase):

A 30 L fermentation was carried out using an *A. oryzae* strain which is a classical amylase expression strain. The fermentation medium and the fermentation conditions are similar to what is described in Bailey and Linko (1990) and Carlsen *et al.* 1995.

### 2. Fermentations for Nuclease coexpression

The coexpression strains for *B*. *pumilus* and *B. amyloliquefaciens* described in section 1.2 were used for fermentation experiments. Fermentation conditions were applied as stated in section 1.1. Fermentation broth was harvested and used for standing time experiments. The coexpressed nuclease was not detectable in SDS PAGE analysis which indicates a low expression level of the nuclease but has a clear effect on the viscosity of the fermentation broth.

### 3. Determination of the viscosity

The viscosity of the fermentation broth as obtained above was determined using a RheolabQC rotation viscosimeter of the company AntonPaar.

With this measurement a stirrer rotates within the liquid which exerts a counterforce on said stirrer. Said counterforce is higher, the higher the viscosity of the solution is. In the present setting a spade is rotated at a constant speed in a beaker containing the fermentation broth. The dynamic viscosity is measured and expressed in the unit mPa*s and transferred in relative viscosity by setting the reference (no nuclease treatment) as 100 %.

### 4. Standing time experiment

At the end of the above 30 L fermentations samples from each supernatant were taken and treated with nuclease at different concentrations (0.1% and 1 %). The viscosity was measured (viscosity between 0 h and 5 h) unless otherwise stated. Said samples were left standing for 72 hours at room temperature without stirring and then the viscosity was measured again. The following results were obtained.

### 4.1 Measurement of samples from B. pumilus fermentation after a standing time of 0 - 5 h and 72 h with and without nuclease treatment

### Measurement after 0 h - 5 h:

1.5 kg fermentation broth from fermentation of *B. pumilus* RH12006 was taken and filled into three 600 mL beakers in portions of 500 g each. Beaker No. 1 was treated with 1 % nuclease supernatant (from strain RH11925 - 3.5 mg/ml NucB). Beaker No. 2 was treated with 0.1 % nuclease supernatant (from strain RH11925 - 3.5 mg/ml NucB). Beaker No. 3 was not treated and served as a control sample. The viscosities were measured and the results are presented in Figure 4.

It was found that shortly after the addition of nuclease a lowering of viscosity compared to the reference sample was obtained (reduction of 30 % compared to referential sample). There was no difference between the two types of dosage which indicates a dosage independent effect for concentrations similar or above 0.1 % (v/v) NucB (3.5 mg/ml).

### Measurement after 72 h:

The beakers described above were left standing for 72 h at room temperature. In contrast to the samples treated with nuclease, the comparative sample showed the formation of slime after 72 hours. Then the viscosity was measured again and the results are presented in Figure 5.

It was found that in the referential sample the viscosity increased significantly in contrast to the samples treated with nuclease. For the NucB treated samples a viscosity was obtained that revealed only 10 % of the viscosity measured for the referential sample. Again there were no differences between the two types of dosage.

### 4.2 Determination of the viscosity of samples from the fermentation with Aspergillus oryzae after standing times of 0 h and 72 h with and without nuclease treatment

Samples of a fermentation of *Aspergillus oryzae* having produced an alpha amylase were aliquoted in 500 g portions at the end of the fermentation and treated either with or without 1 % nuclease NucB (3.5 mg/ml). Due to the fact that viscosity of *Aspergillus oryzae* culture samples are influenced stronger by mechanical shear forces than *Bacillus* culture samples (thixotropic effect) the first time point for measurement was moved to 24 hours to exclude an influence of the initial mixing procedure to the viscosity measurement. The samples were incubated and measured after 24 h and 72 h. Measurements were taken every 20 seconds for five seconds over a time span of 5 minutes.

### Measurement after 24 h:

The results are presented in Figure 6. It could be clearly observed that the thixotropic effect is no longer present in the NucB treated sample whereas the non-treated samples show a higher viscosity and an influence by the stirring procedure applied during measurement. The viscosity of the culture broth was reduced by 15 % compared to the starting point of the referential sample.

### Measurement after 72 h:

Compared to the results received after 24 h standing time a further reduction of 30 % compared to referential strain was observed after 72 h, whereas the thixotropic character of the referential sample is still present. The results are presented in Figure 7.

### 4.3 Determination of the viscosity of samples from the fermentation with Bacillus pumilus coexpression strains after standing times of 0 h and 30 h

Samples of fermentations from *Bacillus pumilus* RH12006 and RH12091 (nuclease coexpression) having produced a xylanase were harvested and the viscosity was measured after standing times of 0 h and 24 h.

### Measurement after 0 h:

The viscosity of both samples was measured continuously over a time span of 90 seconds. It was found that the sample with nuclease coexpression has a significantly lower viscosity than the reference without coexpression. The results are shown in Figure 8.

### Measurement after 30 h:

The samples described above were left standing for 30 h. Then the viscosity was measured and again the sample with coexpression showed significantly lower viscosity. The results are presented in Figure 9.

### 4.4 Determination of the viscosity of samples from the fermentation with Bacillus amyloliquefaciens coexpression after standing times of 0 h and 70 h

Samples of fermentations from *Bacillus amyloliquefaciens* RH11094 and the nuclease coexpression strain *B. amyloliquefaciens* RH12111 having produced a xylanase were harvested and the viscosity was measured after standing times of 0 h and 70 h. The reduction was in a range of 40 to 85 % compared to the referential sample for both time points.

### Measurement after 0 h:

The viscosity of both samples was measured continuously over a time span of 90 seconds. It was found that the sample with nuclease coexpression has a significantly lower viscosity than the reference without nuclease coexpression. The results are shown in Figure 10.

### Measurement after 70 h:

The samples described above were left standing for 70 h. Then again the viscosity was measured and the results are presented in Figure 11. The results were similar as observed for time point 0 h.

### 4.5 Treatment of the slime in the referential sample from Bacillus pumilus with nuclease

In the following the viscosity of the referential sample (*B. pumilus* RH12006) was measured for 30 minutes. After 10 minutes 1 % nuclease NucB (3.5 mg/ml) was added and again the viscosity was recorded for 20 minutes. The results are presented in Figure 12.

It was found that the viscosity decreased significantly after the addition of nuclease (reduction of 85 %). After approximately 70 seconds 50 % of viscosity reduction was already achieved. This indicates that a steady state is reached within minutes and not hours after addition of nuclease NucB.

### 4.6 Treatment of the slime in the referential sample from Bacillus amyloliquefaciens with nuclease

In the following the viscosity of the referential sample was measured for 20 minutes. After 10 minutes 1% nuclease supernatant (from strain RH11925 - 3.5 mg/ml)) was added and again the viscosity was recorded for 10 minutes. The results are presented in Figure 13.

It was found that the viscosity decreased significantly after the addition of nuclease. Also for *B. amyloliquefaciens* a similar effect was observed as seen for *B. pumilus.* The steady state was reached within minutes after addition of nuclease.

## Claims

1. Use of a protein having nuclease activity for reducing the viscosity and/or preventing an increase in viscosity of a fermentation broth, which comprises intact microorganisms after termination of the fermentation process.

2. Method for reducing the viscosity and/or preventing an increase in viscosity of a fermentation broth comprising intact microorganisms after termination of the fermentation process, comprising a step of introducing a protein having nuclease activity into the fermentation broth before recovering the end product.

3. The use of claim 1 or the process of claim 2, wherein the end product of the fermentation process is a microorganism of interest or a target product produced by a microorganism.

4. The use of claim 3 or the process of claim 3, wherein the target product produced is selected from an enzyme, a hormone, an immunoglobulin, a vaccine, an antibiotic, an amino acid, a sugar or a vitamin.

5. The use of claim 1, 3 or 4 or the method of claim 2, 3 or 4, wherein the protein having nuclease activity reduces the thixotropy of the fermentation broth after the end of the fermentation process.

6. The use or the method of any of the preceding claims, wherein the protein having nuclease activity is selected from nucleases, preferably bacterial nucleases, more preferably bacterial nucleases belonging to the Pfam family PF14040, even more preferably NucB or NucA from the group comprising Gram-positive bacteria such as a *Bacillus, Brevibacillus, Clostridium, Enterococcus, Geobacillus, Lactobacillus, Lactococcus, Lysinibacillus, Oceanobacillus, Paenibacillus, Staphylococcus, Streptococcus, Streptomyces,* or *Thermoactinomyces* or Gram-negative bacteria such as an *Acinetobacter, Agrobacterium, Burkholderia, Enterobacter, Erwinia, Escherichia, Lysobacter, Methylomonas, Mesorhizobium, Photobacterium, Pseudomonas, Rhizobium, Serratia,* or *Xenorhabdus,* such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis.*

7. The use or the method of any of the preceding claims, wherein the microorganism is selected from filamentous fungi, yeast or bacteria, preferably from *Actinoplanes, Agrobacterium, Bacillus, Brevibacillus, Clostridium, Enterococcus, Escherichia, Erwinia, Geobacillus, Haemophilus, Lactobacillus, Lactococcus, Lysinibacillus, Oceanobacillus, Paenibacillus, Proteus, Pseudomonas, Rhizobium, Staphylococcus, Streptococcus, Streptomyces, Thermoactinomyces, Xanthomonas, Acremonium, Hyprocrea, Aspergillus, Aureobasidium, Bjerkandera, Ceriporiopsis, Chrysosporium, Coprinus, Coriolus, Cryptococcus, Filibasidium, Fusarium, Humicola, Kluyveromyces, Magnaporthe, Mucor, Myceliophthora, Neocallimastix, Neurospora, Paecilomyces, Penicillium, Phanerochaete, Phlebia, Pichia, Piromyces, Pleurotus, Saccharomyces, Schizophyllum, Streptomyces, Talaromyces, Thermoascus, Thielavia, Tolypocladium, Trametes,* or *Trichoderma.*

8. The use or the method according to claim 7, wherein the filamentous fungus, yeast or bacterium is preferably selected from *Hyprocrea jecorina, Trichoderma reesei, Trichoderma viride, Trichoderma harzianum, Trichoderma koningii, Trichoderma longibrachiatum, Aspergillus niger, Aspergillus oryzae, Aspergillus awamori, Aspergillus fumigatus, Aspergillus foetidus, Aspergillus japonicus, Aspergillus nidulans, Humicola insolens, Humicola grisea, Streptomyces sp., Streptomyces violaceoruber, Bacillus licheniformis, Bacillus amyloliquefaciens, Bacillus clausii, Bacillus pumilus, Bacillus subtilis, Bacillus sp, Bacillus megaterium, Geobacillus stearothermophilus, or Thermotoga maritima.*

9. The use or the method of any of the preceding claims, wherein the nuclease is added at the end of the fermentation process or is produced by expression by a microorganism during fermentation.

10. The use or the method of claim 9, wherein the nuclease is added to the fermentation process at a final concentration of at least 10 mg, at least 1 mg, at least 0.1 mg, at least 0.01 mg, at least 0.001 mg, at least 0.0001 mg nuclease per 100 ml fermentation broth.

11. The use or the method of claim 9, wherein the nuclease is expressed by the microorganism of interest or a microorganism capable of producing a target product or by a separate co-cultivated microorganism.

12. The use or the method of any of claims 1 to 11, wherein at least the following steps are encompassed:
(i) cultivating a microorganism of interest or a microorganism capable of producing a target product,
(ii) adding a nuclease by direct addition or by co-expression of the nuclease by the microorganism of interest or the microorganism capable of producing a target product
(iii) obtaining a fermentation broth comprising a nuclease and a) the microorganism of interest or b) a microorganism capable of producing target product and the target product,
(iv) recovering a) the microorganism of interest or b) the target product.

13. The use or the method of any of claims 1 to 11, wherein at least the following steps are encompassed:
(i) cultivating a microorganism of interest or a microorganism capable of producing a target product,
(ii) obtaining a fermentation broth comprising a microorganism of interest or the target product and the microorganism capable of producing a target product,
(iii) adding a nuclease to the fermentation broth, and
(iv) recovering the microorganism of interest or the target product.

14. The use or the method of any of claims 1 to 11, wherein at least the following steps are encompassed:
(i) cultivating a microorganism of interest or a microorganism capable of producing a target product, and co-cultivating a microorganism capable of producing a nuclease,
(ii) obtaining a fermentation broth comprising a microorganism capable of producing a nuclease and a nuclease, and a) a microorganism of interest or b) a target product and the microorganism capable of producing a target product, and
(iii) recovering a) the microorganism of interest or b) the target product.

15. The use or the method of any of claims 12 to 14, wherein the step of recovering the target product may be carried out within a time interval of one hour to two weeks preferably one hour to ten days, more preferably one hour to eight days, still more preferably one hour to six days, still more preferably one hour to 4 days, still more preferably one hour to two days, still more preferably one hour to 1 day after the end of the fermentation process.
